# EUROPEAN PATENT APPLICATION

(11) **EP 1 939 186 A1**
(43) Date of publication of application: **02.07.2008**
(21) Application number: 06425861.9
(22) Date of filing: 22.12.2006
(51) Int. Cl.: C07D 239/54, A61K 31/513, A61P 35/00

(54) **5-Fluorouracil derivatives and their use for the treatment of cancer**

(71) Applicant: Sulfidris S.r.l., 20121 Milan (IT)
(72) Inventor: Sparatore, Anna, 20146 Milan (IT); Santus, Giancarlo, 20146 Milan (IT); Del Soldato, Piero, 20052 Monza (IT)
(74) Representative: Monti, Rinaldo

(57) **Abstract**

The present invention relates to 5-fluoropyrimidine compounds of formula I 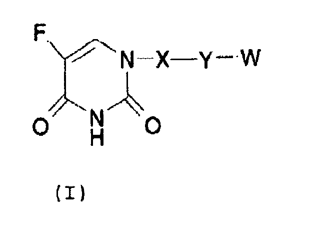 that are useful for preventing, treating or reducing tumoral diseases and pathological conditions involving various cancer forms.

## Description

### Field of the invention

This invention relates to the field of new compounds that are derivatives of 5-fluoropyrimidines whose main biological activity is in the cancer area.

Fluoropyrimidines belong to the family of drugs called antimetabolites. Examples are carmofur, emitefur, tegafur, capecitabine, floxuridine, and fluorouracil (5-FU). Fluoropyrimidines have been used for gastrointestinal cancers (oesophageal, gastric, pancreatic, colon, rectal and anal tumors), head and neck cancers, and breast cancer.

Fluorouracil was the first fluoropyrimidine developed in 1957 based on the observation that tumour cells utilized the base pair uracil for DNA synthesis more efficiently than did normal cells of the intestinal mucosa. It is metabolized intracellularly to its active form, fluorouridine monophosphate (FdUMP). The active form inhibits DNA synthesis by inhibiting thymidylate synthetase and the normal production of thymidine. Effects on RNA (incorporation into RNA and RNA inhibition) occur especially with bolus administration.

The drug is also well known for its adverse effects involving bone marrow, skin, mucous membranes, intestinal tract, central nervous system and cardiovascular system, whereas its cardiotoxicity is a rare but serious side effect, reported in 1-18% of patients [Becker K, Erckenbrecht JF, Haussinger D et al. Cardiotoxicity of the antiproliferative compound fluorouracil. Drugs 1999; 57: 475-484]. The most common symptom of fluorouracil-associated cardiotoxicity is angina-like chest pain. Less common symptoms include cardiac arrythmias, congestive heart failure, myocardial infarction, dilatative cardiomyopathy, cardiogenic shock, cardiac arrest or sudden death syndrome.

Physicians administering any fluoropyrimidine should be aware of the potential for cardiotoxicity and discontinue treatment promptly in patients developing clinical signs of such toxicity.

A significant obstacle for the management of patients with colorectal cancer is intrinsic drug resistance or in patients that respond to chemotherapy, acquired drug resistance. Drug resistance can occur through a variety of mechanisms. These include alterations in drug influx, drug efflux, intracellular metabolic activation, intracellular catabolism, through alterations in the drug's target or through numerous changes downstream of the target including alterations in genes involved in the regulation of the cell cycle, apoptosis or in DNA damage repair.

It is also reported the interaction between fluoropyrimidine and NFkB, a protein localized in the cytoplasm of cells in an inactive form. This protein when activated is translocated into the nucleus where it binds to DNA with subsequent induction of target genes. According to recent knowledge, NF-kB is, among other things, responsible for inducible resistance. Protein products of NF-kB activating genes protect cancer cells against apoptosis - one of the main cytoxic effects of the most efficacious anticancer drugs. The compounds of the present invention, due also to the NFkB block, are proapoptotic agents and they are able to overcome the phenomenon of cellular resistance induced by 5-FU or other antitumoral agents.

### Summary of the invention

This invention relates to novel fluoropyrimidine compounds that comprise in their formula a polysulfurated group and that are useful for treating diseases and pathological conditions involving various cancer forms. The results have been surprising, in that not only the safety was dramatically improved but also the efficacy was sometimes increased as compared with known fluoropyrimidines.

This invention also relates to processes for preparing these compounds and to pharmaceutical compositions containing these compounds.
In particular the polysulfurated groups linked to the 5-fluoropyrimidines are polysulfurated groups containing 2 or more atoms of sulphur, selected from the group comprising organic thiosulfonates or dithiole-thione derivatives such as (5-(p-hydroxyphenyl)-3H-1,2-dithiol-3-thione, or 1,3-dithiol-2-thione-5-carboxylic acid, 3-thioxo-3H-1,2-dithiole-5-carboxylic acid, 3-thioxo-3H-1,2-dithiole-4-carboxylic acid.

### Description of the invention

It is an object of the present invention new 5-fluoropyrimidine derivative compounds of general formula: 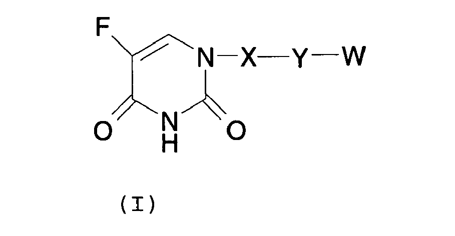 wherein:
X is zero, ~CO-, -(Cₙ)alkyl- straight or branched wherein n is 0-10; or ~CONH-;
Y is zero, - (C_{n'}) alkyl-, ~ (C_{n'}) alkyl-CO-, ~O-(C_{n'}) alkyl-O-, ~OOC- (C_{n'}) alkyl-COO- ; ~ O- (C_{n'}) alkyl-, ~ HN- (C_{n'}) alkyl- , -OOC- (Cₙ ,) alkyl-; - (C_{n'}) alkyl-O-CO- (C_{n''}) alkyl- ; ~(C_{n'})alkyl-CO-0-(C_{n''})alkyl- wherein (C_{n'}) alkyl and (C_{n''})alkyl are straight or branched, and n' and n' ' , the same or different to each other, are 0-10;
W is a polysulfurated group containing 2 or more atoms of sulphur, selected from the group comprising an organic thiosulfonate moiety or a dithiole-thione derivative:
   more in particular, as a further preferred embodiment, wherein W is an organic thiosulfonate
   moiety having the following formula:

      ~S-SO₂-R (II)

      wherein said ~S-SO₂-R is linked to fluoropyrimidine-X-Y~; R is a straight or branched alkyl, such as methyl, ethyl, propyl; alkenyl, alkinyl, alkylaryl, alkenylaryl, alkinylaryl, arylalkyl, arylalkenyl, arylalkinyl or cycloalkyl, cycloalkenyl, cycloalkinyl, or aromatic and/or heterocyclic ring, all substituted or unsubstituted;
   or more in particular, as a further preferred embodiment, wherein W is a dithiole-thione derivative having formula: 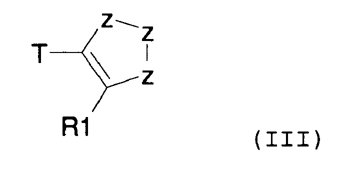
wherein
Z is equal to S (sulphur) provided that at least 1 Z is a thione C=S or a quaternary derivative thereof, and
T is:
~ OOC-; or 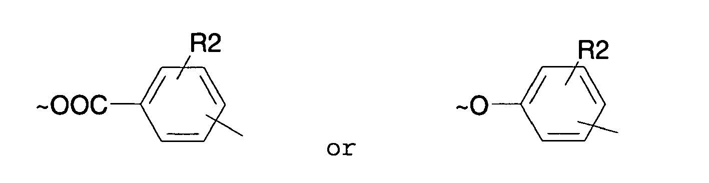 wherein
R1 represents H; -COOH; -NH₂; -OH; -SH;
R2 represents hydrogen, -COOH, alkyl, alkenyl, alkynyl ,aryl, fluoro, chloro, bromo, hydroxyl, alkyloxy, alkenyloxy, aryloxy, acyloxy, amino, alkylamino, arylamino, thio, cyano, nitro, acyl, amido, and a 5 or 6-membered aromatic or nonaromatic ring containing 0, 1, or 2 heteroatoms selected from nitrogen, oxygen, or sulphur;
and salts thereof.

As a further preferred embodiment of the compounds according to general formula (I) of the present invention (Cₙ) alkyl, (C_{n'})alkyl and (C_{n''} alkyl are (CH₂)n_{A} ,(CH2)_{nA}' . (CH₂)_{nA}" respectively, wherein nA, nA' and nA " , the same or different to each other, are 1-10, such as that more preferably X is -(CH₂)_{nA}- and Y is selected from the group comprising -(CH₂)_{nA'}- ~ (CH₂)nA,-CO-, ~O-(CH₂)_{nA'}-O-, ~OOC- (CH₂)_{nA'}-COO- ; ~O-(CH₂)_{nA}--, -HN-(CH₂)_{nA'}-, -OOC- (CH₂) _{nA}' - - (CH₂) _{nA}' -O-Co-(CH₂) _{nA}" - - (CH₂) _{nA}' -Co-o- (CH₂) _{nA}" - wherein nA, nA' and nA" , the same or different to each other, are 1-10.

It is a further object of the present invention the pharmaceutical acceptable salts of compounds of formula (I), such as for example salts with alkaline metals and alkaline earth metals, non-toxic amines and aminoacids, inorganic acids such as hydrochloric acid, phosphoric acid, etc. or organic acids such as fumaric acid, citric acid, tartaric acid, etc.

As a further preferred embodiment of the 5-fluoropyrimidine derivative compounds according to the present invention, are the compounds of general formula (I) wherein the group -Y-W is selected from the group comprising thiosulfonate moieties having the formula: S-(2-carboxyethyl)methanthiosulfonate, S-(2-aminoethyl)methanthiosulfonate and S-(2-hydroxyethyl) methanthiosulfonate.
As a further object of the present invention are compounds according to general formula (I) such as (5-fluoro-2,4-dioxo-3,4-dihydropyrimidin-1(2H)-yl)methyl 3-(methylsulfonylthio)propanoate and 2-(methylsulsonylthio)ethyl 2-(5-fluoro-2,4-dioxo-1,2,3,4-tetrahydropyrimidine-1-carboxamido)acetate. As a further preferred embodiment of the 5-fluoropyrimidine derivative compounds according to the present invention, are the compounds of general formula (I) wherein the polysulfurated group W is selected from the group comprising dithiole-thione derivatives having the formula: 5-(p-hydroxyphenyl)-3H-1,2-dithiol-3-thione, 1,3-dithiol-2-thione-5-carboxylic acid, 3-thioxo-3H-1,2-dithiole-5-carboxylic acid, 3-thioxo-3H-1,2-dithiole-4-carboxylic acid.

As a further object of the present invention is the compound according to general formula (I), which is 4-(5-thioxo-5H-1,2-dithiol-3-yl)phenyl 2-(5-fluoro-2,4-dioxo-1,2,3,4-tetrahydropyrimidine-l-carboxamido)acetate.

According to the present invention it has been found that it is possible to link an organic polysulfurated group to a 5-fluoropyrimidine derivative for treating tumoral diseases. The resulting compounds have good bioavailability, increased safety and maintain good efficacy.

The main advantages of the compounds of the present invention are related to their biological activity also by oral route and to their ability to overcome cellular resistance induced by 5-FU and other antitumoral agents.

When the compounds include at least one asymmetric carbon atom, the products can be used in racemic mixture or in form of single enantiomer.

In the present invention the parent compound is considered in its original form or in a proper modification to allow the chemical manipulation with the polysulfurated group.

The fluoropyrimidine and the polysulfurated group can be linked via different linking groups such as esters, amides, imides, sulfonamides, azo groups, carbamates, carbonates, anhydrides, acetals, thioacetals, etc. The polysulfurated group, i.e. the thiosulfonate moiety or dithiol-thionic derivative can be also directly linked by an ionic bond to the 5-fluoropyrimidine as salt when X=Y=O.

Bi-functional linkers (X and/or Y) known to the expert in the field (such as ethyl, propyl, or butyl diols; di-amines; hydroxy amines, etc.) can be optionally present when they are necessary to link the drug to the polysulfurated group.

Salts of organic thiosulfonates such as, for example, S-(2-carboxyethyl)methanthiosulfonate, S-(2-aminoethyl)methanthiosulfonate with the different fluoropyrimidine above described, are also part of the present invention. Salts of dithiolthiones such as, for example, 1,3-dithiol-2-thione-5-carboxylic acid, 3-thioxo-3H-1,2-dithiole-5-carboxylic acid, 3-thioxo-3H-1,2-dithiole-4-carboxylic acid with the different fluoropyrimidine above described are also part of the present invention.

It is also an object of the present invention the compounds according to general formula (I) wherein the polysulfurated group containing 2 or more atoms of sulphur is in particular a quaternary dithiolthione derivative. Quaternary compounds of dithiolthiones which, according to the present invention, can be linked via different linking groups or directly linked by an ionic bond to the 5-fluoropyrimidine as salt when X=Y=0, can be prepared by several methods such as for example by those described in "Advances in Heterocyclic Chemistry" Katritzky, et al, Vol. 7, 1966, published by Academic Press, pp 39-151.

The anion employed will depend on the properties desired for example solubility, or partial solubility. Example of anions include sulfates, bisulfates, sulfites, bisulfites, halides, i.e. Cl, Br, I, F, etc., phosphates, phosphites, etc., chlorates, etc. Any suitable quaternizing agent may be employed, for example, alkyl halides such as methyl iodide, butyl iodide, butyl bromide, etc.; sulfuric acid derivatives such as R₂SO₄ where R is alkyl, etc., methyl, ethyl, etc. for example (Me)₂ SO₄ ; alkyl thioureas such as methyl thiourea, etc.; sulfonate esters, for example, methansulfonate, methyl p-toluene sulfonates; alkyl phosphates, e.g. (MeO)₃ PO, (EtO)₃ PO, etc. It is to be noted where the anion is polyfunctional, such as difunctional, 2 moles of the dithiolium would be coupled with one mole of the anion.

The thionium compounds are prepared by reacting the 1,2-dithiole-3-thiones with any suitable quaternizing agent at suitable temperatures and times, such as a temperature of from about 20 to 200 °C, but preferably from about 50 to 180 °C, for a period of about 1 to 24 hours, but preferably 3 to 6 hours.

As a further object of the present invention are pharmaceutical compositions comprising at least one compound of the above said 5-fluoropyrimidine derivative compounds (according to the present invention as for general formula (I), and the preferred compounds as described above) including salts thereof, as an active ingredient, moreover, as a further object of the present invention, in combination with pharmaceutically acceptable adjuvant(s) or carrier(s).

It is a further object of the present invention the use of the above said 5-fluoropyrimidine derivative compounds as for general formula (I), and the preferred compounds as described above, as a medicament.

As a further object of the present invention is the use of compounds according to the present invention, as for general formula (I) and the preferred compounds as described above, for the preparation of a pharmaceutical composition, and therefore the corresponding method, for preventing, treating or reducing tumoral diseases also in combination with other antitumoral agents.

The compounds of the present invention can be administered in the form of any pharmaceutical formulation, the nature of which will depend upon the route of administration and the nature of the disease to be treated. These pharmaceutical compositions can be prepared by conventional methods, using compatible, pharmaceutically acceptable excipients or vehicles. Examples of such compositions include capsules, tablets, syrups, powders and granulates for the preparation of extemporaneous solutions, injectable preparations, rectal, nasal, ocular, vaginal etc.

A preferred route of administration is the oral route.

It is a further object of the present invention the process of the synthesis of 5-fluoropyrimidine derivative compounds, as for general formula (I) and preferred compounds as described above, said process comprising the reaction of 5-fluoropyridine with polysulfurated group W comprising thiosulfonate moiety or dithiol-thione derivate, said polysulfurated group W and the 5-fluoropyrimidine eventually modified with X and/or Y, being said W, X and Y as defined above.

It is a further object of the present invention the use of 5-fluoropyrimidine derivative compounds as for general formula (I), and the preferred compounds as described above, for preventing, treating or reducing tumoral diseases, also in combination with other antitumoral agents, as well as the method for preventing, treating or reducing tumoral diseases, said method comprising the use of 5-fluoropyrimidine derivative compounds as for general formula (I), and the preferred compounds as described above.

The following non-limitative examples further describe and enable an ordinary skilled in the art to make and use the invention.

### EXAMPLE 1. Synthesis of (5-fluoro-2,4-dioxo-3,4-dihydropyrimidin-1(2H)-yl)methyl 3-(methylsulfonyl thio)propanoate.

### Step 1: Preparation of 3-methanesulfonylsulfanyl-propionic acid.

Sodium methanthiosulfonate (1.5 g; 11.18 mmol) and 3-bromopropionic acid (900 mg; 5.88 mmol) 97% are dissolved in dimethylformamide (DMF, 9.05 ml). The reaction is performed at 60° C, stirring under nitrogen for 4 hours. The solution is evaporated to dryness and the residue is dissolved in water, acidified with 10 ml of a 2N KHSO₄ and washed with ethyl acetate. The organic phase is extracted with cold 2N KHSO₄, then with brine and finally dried on anhydrous sodium sulphate and evaporated to dryness to obtain an oily yellow product.

### Step 2.

180 mg (1.38 mmol) of 5-FU are added to 0.23 ml of an aqueous solution of formaldehyde (37%) and the mixture is kept under stirring for 45 min. at 60°C. Water is removed, after cooling at room temperature, and the obtained viscous liquid compound is dissolved in 11.5 ml of anhydrous acetonitrile.

To this solution are added 1,2 eq. of dicyclohexylcarbodiimide (DCC), catalytic amount of 4-dimethylaminopyridine (DMAP) and 1.2 eq of the compound prepared in step 1.

The reaction is performed stirring under nitrogen at room temperature for 4 hours. At the end of the reaction, the dicyclohexylurea is filtered and the organic solution is washed with 1N HCl, a 5% solution of NaHCO₃ and a saturated solution of NaCl. The organic phase is dried on anhydrous sodium sulphate and evaporated to dryness. The residue is purified by chromatography on silica gel using as eluting mixture methylene chloride with methanol up to 3%. The obtained product, washed with ether, has a melting point of 149-152° C.

### EXAMPLE 2. Synthesis of 2-(5-fluoro-2,4-dioxo-1,2,3,4-tetrahydropyrimidine-1-carboxamido)acetic acid.

500 mg (3.85 mmol) of 5-FU are suspended in 1.7 ml of anhydrous pyridine and 0.93 ml of ethylisocyanato acetate (2eq). The reaction is performed stirring under nitrogen at 90 °C for 3 hours. 100 ml of a mixture methylene chloride/methanol (95/5) are added, after cooling at room temperature. The organic phase is washed with 1N HCl and then with a saturated solution of NaCl. The organic phase is dried on anhydrous sodium sulphate and evaporated to dryness. The obtained product, washed with ether, has a melting point of 151-154 °C.

900 mg (2,32 mmol) of this product are suspended in 6.2 ml of concentrated HCl heating at 70 °C for 30 minutes. The precipitate, obtained after cooling at room temperature and filtration, is washed with water, then with cold ethanol and finally with ether. The obtained compound melts with decomposition after 150 °C.

### EXAMPLE 3. Synthesis of 4-(5-thioxo-5H-1,2-dithiol-3-yl)phenyl 2-(5-fluoro-2,4-dioxo-1,2,3,4-tetrahydro pyrimidine-1-carboxamido)acetate.

*Step* 1: Preparation of 5-(p-hydroxyphenyl)-3H-1,2-ditiol-3-thione.

To 280 mmol of sulphur, 40 mmol of anethole in 20 ml of dimethylacetamide were added. After heating at 145 °C for 6 hours, 2.5 g of anethole dithiolethione (ADT) were obtained. The product, washed with ether, was crystallized by ethyl acetate: melting point 110-111 °C. Then 1.5g of ADT were mixed with 7.5g of pyridine HCl and the mixture was heated for 25 minutes at 215 °C. After cooling, 1N HCl in excess was added and the precipitate was filtered, washed and crystallized from ethanol. The obtained compound, 5-(p-hydroxyphenyl)-3H-1,2-ditiol-3-thione, melted at 191-192 °C.

### Step 2

200 mg (0.87 mmol) of the compound of example 2 are dissolved in 10 ml of anhydrous tetrahydrofuran (THF). To this solution are added 197 mg of the compound prepared in step 1 (0.87 mmol), (215.4 mg, 1,2 eq.) of dicyclohexylcarbodiimide (DCC), catalytic amount of 4-dimethylaminopyridine (DMAP). The reaction is performed stirring under nitrogen at room temperature for 30 minutes. At the end of the reaction, the dicyclohexylurea is filtered and the organic solution is washed with methanol and ether. The product is crystallized by THF and the crystals are washed with a mixture of ether/THF (8/2). The obtained product, washed with ether, has a melting point of 196-200° C.

### EXAMPLE 4. Synthesis of 2-(methylsulfonylthio)ethyl 2-(5-fluoro-2,4-dioxo-1,2,3,4-tetrahydropyrimidine-1-carboxamido)acetate.

*Step* 1: Synthesis of methanethiosulfonic acid S-(2-hydroxyethyl) ester.

A solution of CH₃SO₂Cl (5.9 g) in ethanol (9.2 ml) is added dropwise to a refrigerated (-15°C) solution of Na₂S (46.98 mmol) in ethanol(34.5 ml).

The reaction mixture is stirred at room temperature for 12 hours. After filtration and crystallization from ethanol, sodium methanthiosulfonate, as a white solid is obtained. The sodium methanthiosulfonate (2.5 g; 18.64 mmol) is dissolved in 30 ml of ethanol and a solution of 2-bromoethanol (2.6 ml; 37.28 mmol) in ethanol (6 ml) is added dropwise. The solution is heated at 100 °C for 8 hours under nitrogen. The mixture is filtered, the solution is evaporated to dryness and the residue is dissolved in CHCl₃ and extracted with water.

The aqueous solution is evaporated to dryness, tetrahydrofuran (THF) is added to the residue and the obtained suspension is filtered. The THF solution is evaporated and methanethiosulfonic acid S-(2-hydroxyethyl) ester as an oily yellow product is obtained.

### Step 2:

250 mg (1.08 mmol) of the compound described in example 2 are dissolved in 6 ml of anhydrous tetrahydrofuran (THF). To this solution are added in following order: 169 mg of the compound prepared in step 1 (1.08 mmol), (270 mg, 1,2 eq. of dicyclohexylcarbodiimide (DCC), catalytic amount of 4-dimethylaminopyridine (DMAP). The reaction is performed stirring under nitrogen at room temperature for 30 minutes. At the end of the reaction, the dicyclohexylurea is filtered and the organic solution, after evaporation of the solvent, is washed with methanol and ether. The obtained product, washed with ether, has a melting point of 118-120 °C.

### EXAMPLE 5. Biological activity

The compound described in example 4 has been tested in vitro on colon tumour cells HCT 116 and DLD 1. MTT assay was used for toxicity study (spectrophotometric measurement of cell viability by mitochondrial dehydrogenase).

The compound potently inhibit growth of both colon tumour cells in a range of concentrations 1-100 µM.

### EXAMPLE 6. Biological activity on resistant cells

Cisplatin resistant human ovarian cancer cells were treated with compounds of example 1 and 4 in comparison with 5-FU at various concentrations (1-1000 µM) for 24, 48 or 72 hours. MTD assay was used for toxicity study.

The compounds of example 1 and 4 have a biological activity superior to 5-FU in the range of concentrations tested.

## Claims

1. Compounds of general formula: 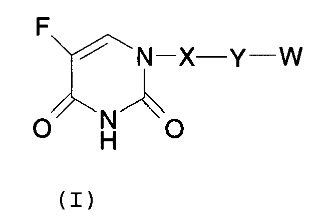 wherein:
X is zero, ~CO-, -(Cₙ)alkyl- straight or branched wherein n is 0-10; or ~CONH-;
Y is zero, - (C_{n'}) alkyl-, ~ (C_{n'}) alkyl-CO-, -O-(C_{n'}) alkyl-O-,
~OOC-(C_{n'})alkyl-COO- ; ~O-(C_{n'})alkyl-, ~HN-(C_{n'})alkyl- , -OOC- (C_{n'}) alkyl-; - (C_{n'}) alkyl-O-CO- (C_{n''}) alkyl-; ~(C_{n'})alkyl-CO-O-(C_{n"})alkyl- wherein (C_{n'})alkyl and (C_{n''})alkyl are straight or branched, and n' and n' ' , the same or different to each other, are 0-10;
W is a polysulfurated group containing 2 or more atoms of sulphur, selected from the group comprising an organic thiosulfonate moiety or a dithiole-thione derivative,
and salts thereof.

2. Compounds of general formula (I) according to claim 1, wherein W is an organic thiosulfonate moiety having the following formula:
~S-SO₂ (II)
wherein said ~S-SO₂-R is linked to fluoropyrimidine-X-Y~; R is a straight or branched alkyl, such as methyl, ethyl, propyl; alkenyl, alkinyl, alkylaryl, alkenylaryl, alkinylaryl, arylalkyl, arylalkenyl, arylalkinyl or cycloalkyl, cycloalkenyl, cycloalkinyl, or aromatic and/or heterocyclic ring, all substituted or unsubstituted.

3. Compounds of general formula (I) according to claim 1, wherein W is a dithiole-thione derivative having formula: 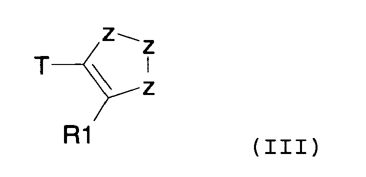 wherein
Z is equal to S (sulphur) provided that at least 1 Z is a thione C=S or a quaternary derivative thereof, and
T is:
~COO-; or 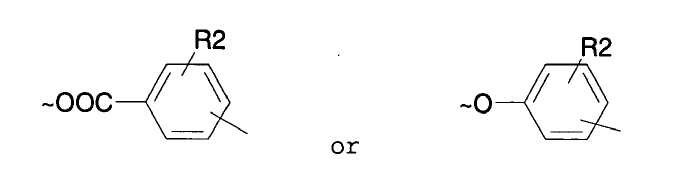 R1 represents H; -COOH; -NH₂; -OH; -SH;
R2 represents hydrogen, -COOH, alkyl, alkenyl, alkynyl ,aryl, fluoro, chloro, bromo, hydroxyl, alkyloxy, alkenyloxy, aryloxy, acyloxy, amino, alkylamino, arylamino, thio, cyano, nitro, acyl, amido, and a 5 or 6-membered aromatic or nonaromatic ring containing 0, 1, or 2 heteroatoms selected from nitrogen, oxygen, or sulphur.

4. Salts of compounds of general formula (I) according to claim 1, wherein said salts are pharmaceutical acceptable salts of compounds of formula (I).

5. Salts according to claim 4, said salts comprising salts of compounds of formula (I) with: alkaline metals and alkaline earth metals, non-toxic amines and aminoacids, inorganic acids comprising hydrochloric acid, phosphoric acid or organic acids comprising fumaric acid, citric acid, tartaric acid.

6. Compound of general formula (I) according to claim 1, wherein the group -Y-W is selected from the group comprising thiosulfonate moieties having the formula: S-(2-carboxyethyl)methanthiosulfonate, S-(2-aminoethyl)methanthiosulfonate and S-(2-hydroxyethyl) methanthiosulfonate.

7. Compound of general formula (I) according to claim 1, wherein the polysulfurated group W is selected from the group comprising dithiole-thione derivatives having the formula: 5-(p-hydroxyphenyl)-3H-1,2-dithiol-3-thione, 1,3-dithiol-2-thione-5-carboxylic acid, 3-thioxo-3H-1,2-dithiole-5-carboxylic acid, 3-thioxo-3H-1,2-dithiole-4-carboxylic acid.

8. Compound of general formula (I) according to claim 1, wherein said compound is (5-fluoro-2,4-dioxo-3,4-dihydropyrimidin-1(2H)-yl)methyl 3-(methyl sulfonylthio)propanoate.

9. Compound of general formula (I) according to claim 1, wherein said compound is 2-(methylsulfonylthio) ethyl 2-(5-fluoro-2,4-dioxo-1,2,3,4-tetrahydro pyrimidine-1-carboxamido)acetate.

10. Compound of general formula (I) according to claim 1, wherein said compound is 4-(5-thioxo-5H-1,2-dithiol-3-yl)phenyl 2-(5-fluoro-2,4-dioxo-1,2,3,4-tetrahydropyrimidine-1-carboxamido) acetate.

11. Pharmaceutical composition comprising at least one compound of general formula (I) as claimed in one of the claims 1-10 as an active ingredient and eventually one or more pharmaceutically acceptable adjuvant(s) or carrier(s).

12. Compound of general formula (I) as claimed in one of the claims 1-10 for use as a medicament.

13. Use of a compound of general formula (I) as claimed in one of the claims 1-10 for the preparation of a pharmaceutical composition for preventing, treating or reducing tumoral diseases.

14. Process for the synthesis of compounds of general formula (I) according to claim 1, said process comprising the reaction of 5-fluoropyridine with polysulfurated group W comprising thiosulfonate moiety or dithiol-thione derivate, said polysulfurated group W and the 5-fluoropyrimidine eventually modified with X and/or Y, being said W, X and Y as defined in claim 1.
